# EUROPEAN PATENT APPLICATION

(11) **EP 4 755 316 A1**
(43) Date of publication of application: **10.06.2026**
(21) Application number: 24849047.6
(22) Date of filing: 25.07.2024
(51) Int. Cl.: A61B 10/00, A61N 5/06

(54) **BRAIN ACTIVITY MEASURING DEVICE**

(30) Priority: 28.07.2023 JP 2023123748
(71) Applicant: UploadedMind Inc., Tokyo 102-0082 (JP)
(72) Inventor: TASHIRO, Kengo, Tokyo 102-0082 (JP); KAGIMOTO, Tadahisa, Tokyo 106-0041 (JP); MATSUO, Yutaka, Tokyo 113-0024 (JP)
(74) Representative: Mathys & Squire
(86) International application number: PCT/JP2024/026621
(87) International publication number: WO 2025/028393

(57) **Abstract**

[Problem] The purpose of the present invention is to provide a brain activity measuring device that is capable of measuring brain activity over a wide region.

[Solution] The brain activity measuring device comprises: a brain diagnosis probe; a detection signal reception unit for receiving a detection signal from the brain diagnosis probe; and a signal analysis unit for analyzing the detection signal received by the detection signal reception unit and obtaining measurement information for measuring brain activity. The brain diagnosis probe has multiple sheet-shaped probe units, and each of the multiple sheet-shaped probe units is placed inside the skull via a hole prepared in the skull of the living organism in question.

## Description

### TECHNICAL FIELD

The present invention relates to a brain activity measurement device. More specifically, the present invention relates to a brain activity measurement device capable of measuring brain activity over a wide area and suitable for brain activity measurement using calcium-sensitive fluorescent proteins or optogenetics.

### BACKGROUND ART

Japanese Patent No. 3718500 describes a probe and device for measuring hemodynamic and oxygen processing characteristics of the brain. This device uses a probe having optical fibers. Accordingly, this device can measure brain activity only in the areas where the optical fibers are laid.

### PRIOR ART DOCUMENTS

### Patent Literatures

[Patent Literature 1] Japanese Patent No. 3718500

### SUMMARY OF INVENTION

### Technical Problem

An object of the present invention is to provide a brain activity measurement device capable of measuring brain activity over a wide area. Another object of the present invention is to provide a brain activity measurement device 1 suitable for brain activity measurement using calcium-sensitive fluorescent proteins or optogenetics. The present invention aims to solve at least one of the above problems.

### Means for solving the technical problem

The above problems are basically solved based on the finding that, by using a plurality of sheet-shaped probe units, the sheet-shaped probe units can be laid inside the brain, and as a result, brain activity can be measured over a wide area, which is also suitable for brain activity measurement using calcium-sensitive fluorescent proteins or optogenetics.

A brain activity measurement device 1 described in this specification comprises a brain diagnostic probe 3, a detection signal receiving unit 5, and a signal analysis unit 7. The brain diagnostic probe 3 has a plurality of sheet-shaped probe units 9. Each of the plurality of sheet-shaped probe units 9 is placed inside a skull 11 via a hole 13 formed in the skull 11 of a subject. The detection signal receiving unit 5 is an element for receiving a detection signal from the brain diagnostic probe 3. The signal analysis unit 7 is an element for analyzing the detection signal received by the detection signal receiving unit 5 and obtaining measurement information for measuring brain activity.

Preferably, each of the plurality of sheet-shaped probe units has undulation corresponding to at least one of, or both, undulation of brain parenchyma and vascular structure at the site where each sheet-shaped probe unit is laid. In a preferred example of the brain activity measurement device 1, the plurality of sheet-shaped probe units 9 have a shape configured to be laid on at least one of a subdural space, a subarachnoid space, a ventricular wall, or a brain sulcus of the subject.

In a preferred example of the brain activity measurement device 1, the plurality of sheet-shaped probe units 9 have a shape covering from 1% to 90%, inclusive, of the subject's brain region.

In a preferred example of the brain activity measurement device 1, at least one of the plurality of sheet-shaped probe units 9 comprises a first light source 15, a first detector 17, and a first light control unit 19 for controlling light output from the first light source 15. The first light source 15 is preferably used to excite a luminescent substance contained in the brain.

In a preferred example of the brain activity measurement device 1, at least one of the plurality of sheet-shaped probe units 9 has a second light source 21 having a wavelength different from the wavelength of the first light source 15. The second light source 21 is used for treating the brain of the subject.

In a preferred example of the brain activity measurement device 1, the signal analysis unit 7 analyzes brain activity of the subject using machine learning to obtain intention information of the subject. This brain activity measurement device 1 further comprises an intention information output unit 23 for outputting the intention information.

In a preferred example of the brain activity measurement device 1, the signal analysis unit 7 analyzes activity of neurons of the subject using machine learning.

In a preferred example of the brain activity measurement device 1, the neurons of the subject emit light in response to a predetermined light stimulus by gene introduction or introduction of a luminescent substance.

In a preferred example of the brain activity measurement device 1, the device further comprises a wireless output unit 25 for wirelessly outputting the detection signal or the measurement information to outside of the subject.

In a preferred example of the brain activity measurement device 1, the device further comprises a deep brain stimulation electrode 31, a power supply unit 33 for supplying power to the deep brain stimulation electrode 31, and a supply power control unit 35 for controlling the power supplied by the power supply unit 33.

### Advantageous Effects of Invention

The brain activity measurement device of the present invention can be laid over a wide area of the brain by using a plurality of sheet-shaped probe units, and as a result, brain activity can be measured over a wide area, making it suitable for brain activity measurement using calcium-sensitive fluorescent proteins or optogenetics.

### BRIEF DESCRIPTION OF DRAWINGS

[FIG. 1] FIG. 1 is a block diagram for explaining the brain activity measurement device.
[FIG. 2] FIG. 2 is a conceptual diagram showing an example of laying the brain diagnostic probe.
[FIG. 3] FIG. 3 is a diagram showing examples of the sheet-shaped probe unit.
[FIG. 4] FIG. 4 is a conceptual diagram showing a state where the first detector detects fluorescence emitted by a fluorescent substance.
[FIG. 5] FIG. 5 is a conceptual diagram showing a design example of a circuit on a sheet-shaped probe unit having the first and second light sources.

### DESCRIPTION OF EMBODIMENTS

Hereinafter, embodiments for carrying out the present invention will be described with reference to the drawings. The present invention is not limited to the embodiments described below, and includes modifications that are obvious to those skilled in the art from the following embodiments.

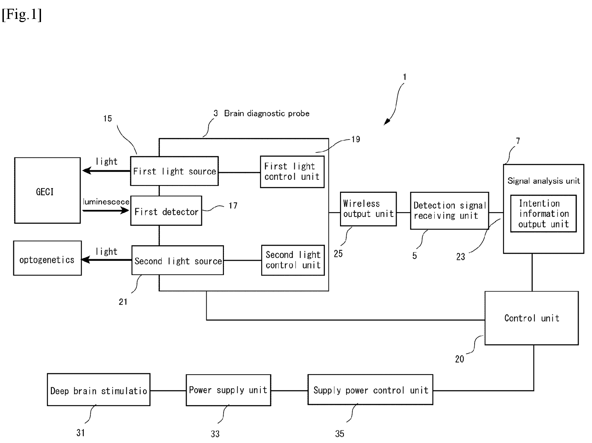

FIG. 1 is a block diagram for explaining the brain activity measurement device. As shown in FIG. 1, the brain activity measurement device 1 comprises a brain diagnostic probe 3, a detection signal receiving unit 5, and a signal analysis unit 7. The brain diagnostic probe 3 is inserted into the skull (the space between the skull and the brain surface, the subdural space) and is an element for performing brain diagnosis. The brain diagnostic probe 3 is known, as described, for example, in Japanese Patent No. 3718500 and Japanese Patent No. 5224482.

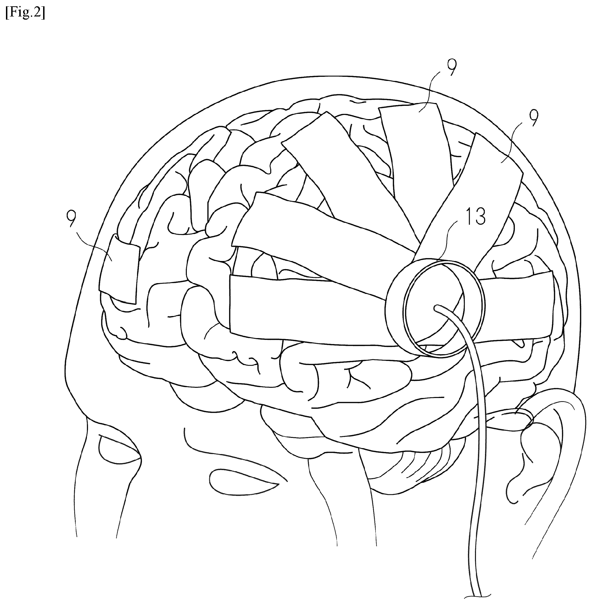

FIG. 2 is a conceptual diagram showing an example of laying the brain diagnostic probe. As shown in FIG. 2, the brain diagnostic probe 3 has a plurality of sheet-shaped probe units 9. Each of the plurality of sheet-shaped probe units 9 is placed inside a skull 11 via a hole 13 formed in the skull 11 of a subject. The number of holes 13 may be one, or two or more. The hole 13 may be formed on a lateral side of the skull, a posterior side of the skull, or an upper part of the skull. In the example of FIG. 2, two holes are formed. Examples of the number of sheet-shaped probe units 9 include 2 to 100, or 3 to 50, or 4 to 20. Examples of the subject include animals, and preferred examples of the subject include humans or mammals other than humans. Among these, humans are preferred as the subject.

Preferably, the plurality of sheet-shaped probe units 9 have a shape covering from 1% to 90%, inclusive, of the subject's brain region. Here, the brain region means a region between the cerebrum and the skull. The brain region covered by the plurality of sheet-shaped probe units 9 may be 5% to 90%, 10% to 90%, 15% to 80%, 20% to 50%, 20% to 40%, or 30% to 70%.

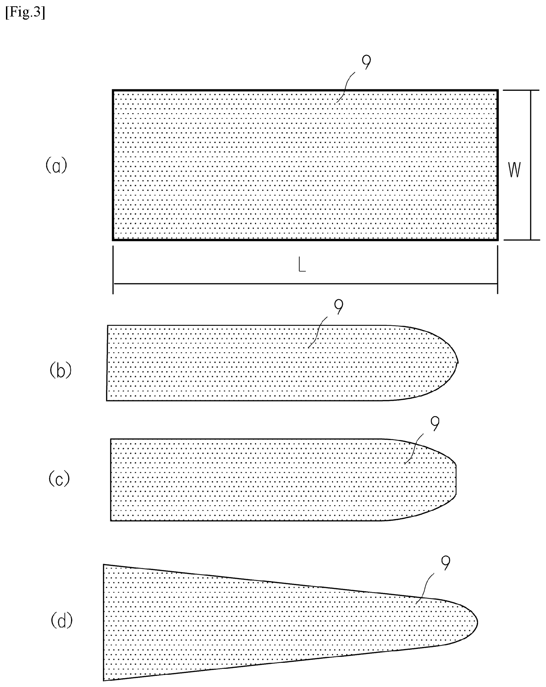

FIG. 3 is a diagram showing examples of the sheet-shaped probe unit. FIG. 3(a) shows a sheet-shaped probe having a rectangular shape, FIG. 3(b) shows a sheet-shaped probe having a shape with a rounded tip, FIG. 3(c) shows a sheet-shaped probe having a shape with chamfered corners at the tip, and FIG. 3(d) shows a sheet-shaped probe having a shape that becomes narrower toward the tip with a rounded end. As shown in FIG. 3, the shape of the sheet-shaped probe unit 9 may be strip-shaped (rectangular) or may have a shape with a tapered tip for easy insertion into the brain. The shape of the sheet-shaped probe unit 9 may have a rounded tip or chamfered corners at the tip. The sheet-shaped probe unit 9 is preferably a flexible sheet. Furthermore, since the sheet-shaped probe unit 9 is intended to be laid between the skull and the brain surface, and particularly preferably laid along the cerebral cortex, the sheet-shaped probe unit 9 is preferably highly biocompatible. The sheet-shaped probe unit 9 preferably has a certain degree of flexibility and rigidity, has a property of adhering due to moisture, but has a property of not adhering to the brain surface and being removable from the brain surface.

Preferably, each of the plurality of sheet-shaped probe units has undulation corresponding to at least one of, or both, undulation of brain parenchyma and vascular structure at the site where each sheet-shaped probe unit is laid. The brain includes various areas such as the frontal lobe, parietal lobe, temporal lobe, and occipital lobe, and the sheet-shaped probe unit 9 may be laid on each of these regions. The sheet-shaped probe unit 9 may also have a shape adapted to the region where it is laid. Examples of the width W of the sheet-shaped probe unit 9 (the length at the widest part) include 0.5 cm to 6 cm, 1 cm to 5 cm, 1 cm to 4 cm, 1 cm to 3 cm, or 2 cm to 4 cm. Examples of the length L of the sheet-shaped probe unit 9 (the length at the longest part) include 2 cm to 20 cm, 4 cm to 15 cm, or 5 cm to 10 cm.

For example, each of the plurality of sheet-shaped probe units 9 is designed for a predetermined site where it is to be laid. For example, each sheet-shaped probe unit 9 preferably has a shape and properties suitable for being laid on at least one of the cerebral cortex, ventricles, subdural space, subarachnoid space, ventricular wall, or brain sulcus of the subject. Since each of the plurality of sheet-shaped probe units 9 has undulation corresponding to at least one of, or both, undulation of brain parenchyma and vascular structure at the site where each sheet-shaped probe unit is to be laid, it can be brought as close to and in contact with the brain parenchyma as possible. For example, the undulation and vascular structure of the brain parenchyma may be examined in advance using CT or MRI, and undulation corresponding to those shapes may be custom-made. In this example, the method may include a brain surface shape acquisition step, which is a step of acquiring at least one of, or both, undulation of brain parenchyma and vascular structure (brain surface undulation information) at the site including the site where the sheet-shaped probe unit 9 is to be laid, and a surface-adjusted sheet-shaped probe unit production step of obtaining a sheet-shaped probe unit 9 having a surface shape based on the brain surface undulation information. The brain surface shape acquisition step may use a method capable of acquiring brain shape information, such as CT or MRI described above. The acquired brain surface undulation information may be appropriately stored in a storage unit. Next, in the surface-adjusted sheet-shaped probe unit production step, the brain surface undulation information is read from the storage unit, and the surface shape of the sheet-shaped probe unit is adjusted. For example, after installing elements and circuits necessary for functioning as a sheet-shaped probe unit on a flexible substrate, its surface is coated with resin to produce the sheet-shaped probe unit 9. When coating the flexible substrate with resin, the surface shape of the sheet-shaped probe unit 9 may be adjusted based on the brain surface undulation information to obtain a sheet-shaped probe unit 9 whose surface shape corresponds to the undulation and vascular structure of brain parenchyma. Such processing can be achieved using, for example, a 3D printer. Of course, after forming a resin layer on the flexible substrate, the resin layer may be processed based on the brain surface undulation information to obtain a sheet-shaped probe unit 9 whose surface shape corresponds to the undulation and vascular structure of brain parenchyma.

The sheet-shaped probe may use a flexible printed circuit board. From the viewpoint of biocompatibility, a flexible printed circuit board with copper foil attached to its surface is preferred. Examples of such flexible printed circuit boards are described in Japanese Patent No. 7194857 and Japanese Patent No. 7164752. With a flexible printed circuit board, the light source, detector, control unit (and storage unit) described below can be installed on the substrate while maintaining flexibility. However, the flexible printed circuit board preferably has its entirety coated and has a coating layer. This is to prevent light sources and other elements from being left behind in the brain or damaging the brain surface. The coating layer is preferably transparent or translucent to transmit light.

In a preferred example of the brain activity measurement device 1, at least one of the plurality of sheet-shaped probe units 9 (preferably all of the sheet-shaped probe units 9) has a first detector 17. The sheet-shaped probe unit 9 may have a first light source 15, a first detector 17, and a first light control unit 19 for controlling light output from the first light source 15. The first detector 17 is used to detect a signal related to brain activity. The first light source 15 may be used to excite a luminescent substance contained in the brain. In this example, for instance, a luminescent substance is introduced into the brain of the subject. As such, it is preferable that the neurons of the subject emit light in response to a predetermined light stimulus by gene introduction or introduction of a luminescent substance. Examples of the luminescent substance include fluorescent proteins. Examples of fluorescent proteins include green fluorescent protein (e.g., GFP), red fluorescent protein (e.g., DsRed), and genetically encoded calcium indicators (GECI). The genetically encoded calcium indicators are fluorescent proteins having calcium sensitivity. For example, a luminescent substance (GECI) is introduced into the neurons of the subject. Examples of GECI include GCaMP proteins (GCaMP3 protein, GCaMP6s protein, GCaMP7 protein, RCaMP1) and aequorin. However, GECI discovered in the future may also be used. The method for introducing GECI is known, as described, for example, in Japanese Patent No. 5854686. When a GECI is introduced, the GECI emits light in response to calcium fluctuations when the brain is activated. For example, the GECI contained in neurons of the brain emits light. In this way, the method of measuring brain function using luminescent substances can measure brain function more accurately and rapidly compared to methods such as functional near-infrared spectroscopy and optical topography.

Each sheet-shaped probe unit 9 may have a plurality of first light sources 15. The first light control unit 19 controls the light intensity and ON/OFF of the first light source 15. The first light control unit 19 may receive a control command from the control unit 20 and control the first light source 15 according to the control command. The control unit 20 may be implemented by, for example, a computer or processor. The control unit 20 may output information for controlling the first light source 15 to the first light control unit 15 based on the measurement information obtained by the signal analysis unit 7. The expressions "control unit 19" and "control unit 20" are functional, and they may be physically the same element. Furthermore, the first light control unit 19 may store a program and control the first light source 15 based on instructions from the program. The first light control unit 19 may control a plurality of first light sources 15. The plurality of first light sources 15 may be, for example, installed at equal intervals on the sheet-shaped probe unit 9. Examples of the first light source 15 include light-emitting diodes and LEDs. The output of the first light source 15 is preferably at an intensity that does not damage the brain. The wavelength of the light output from the first light source 15 is preferably a wavelength corresponding to the luminescent substance. By using the first light source 15, the fluorescent substance can be excited. An example of the first light source 15 is visible light, and it is preferably a light source that emits light with a wavelength of 400 nm to 600 nm. The light from the first light source 15 may be, for example, pulsed light or continuous light.

An example of the first detector 17 is an optical sensor. The first detector 17 may measure a physical quantity other than light related to brain activity. The first detector 17 may detect emission from the luminescent substance. The first detector 17 may detect reflected light after the first light source 15 irradiates light, or may detect light emitted by the luminescent substance in response to light irradiated from the first light source 15. An example of the first detector 17 is a photodiode (PD). Each sheet-shaped probe unit 9 preferably has a plurality of first detectors 17.

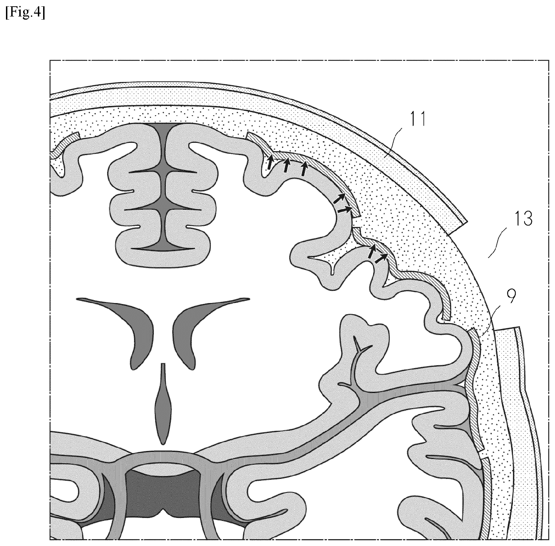

FIG. 4 is a conceptual diagram showing a state where the first detector detects fluorescence emitted by a fluorescent substance. In this example, light output from the first light source 15 excites GECI introduced into neurons. Then, the first detector 17 detects the light (arrow) emitted by the excited GECI.

The optical signal detected by the first detector 17 is converted into a detection signal and output to the detection signal receiving unit 5. The processing of converting the optical signal into a detection signal and the processing of outputting the detection signal to the detection signal receiving unit 5 may be performed by, for example, the first detector 17. A normal photodiode can perform these processes. The detection signal receiving unit 5 is connected to receive signals from the brain diagnostic probe 3. The detection signal receiving unit 5 may be installed inside the brain together with the brain diagnostic probe 3. Alternatively, the detection signal receiving unit 5 may be provided outside the brain. For example, the brain activity measurement device 1 may further comprise a wireless output unit 25 for wirelessly outputting the detection signal to outside of the subject. The wireless output unit 25 may wirelessly output the detection signal, and the detection signal receiving unit 5 may receive the detection signal.

The detection signal receiving unit 5 is an element for receiving a detection signal from the brain diagnostic probe 3. When the detection signal receiving unit 5 and the brain diagnostic probe 3 are connected by wire, the detection signal receiving unit 5 may receive the detection signal output from the brain diagnostic probe 3 via a wire, such as electrical or optical signals. When the detection signal is output as a wireless signal, the detection signal receiving unit 5 has an element for receiving wireless signals, such as an antenna, and may receive the detection signal that is a wireless signal.

The signal analysis unit 7 is an element for analyzing the detection signal received by the detection signal receiving unit 5 and obtaining measurement information for measuring brain activity. The signal analysis unit 7 may be implemented by a computer or processor.

A computer comprises an input unit, an output unit, a control unit, an arithmetic unit, and a storage unit, and each element is connected by a bus or the like to enable information exchange. For example, the storage unit may store programs and various types of information. When predetermined information is input from the input unit, the control unit reads a program stored in the storage unit. The control unit appropriately reads information stored in the storage unit and transmits it to the arithmetic unit. The control unit also appropriately transmits input information to the arithmetic unit. The arithmetic unit performs arithmetic processing using the received various types of information and stores the result in the storage unit. The control unit reads the arithmetic result stored in the storage unit and outputs it from the output unit. In this manner, various processes and steps are executed. The elements that execute these various processes are the various units and means. The computer may have a processor, and the processor may realize various functions and steps. The computer may be standalone. Some functions of the computer may be distributed between a server and a terminal. In that case, the server and the terminal are preferably connected via a network such as the Internet or an intranet to enable information exchange. The computer may comprise a processor and a memory coupled to the processor. The memory may store instructions that, when executed by the processor, cause the computer to perform various steps or function as various elements. The computer may be configured to construct a learning model by providing various training data and realize various calculations through machine learning. In this case, the computer may execute various analyses using a learning model created by machine learning and deep learning of AI (artificial intelligence). In this way, the accuracy of machine learning is improved.

For example, the signal analysis unit 7 has a learning model constructed from past detection signals and brain activity. The signal analysis unit 7 may analyze the detection signal using the learning model to obtain measurement information related to real-time brain activity. The signal analysis unit 7 may also store past measurement information of the subject and obtain real-time measurement information by comparison with the past measurement information. Examples of the measurement information include the degree and progression of brain diseases. Brain diseases mean diseases caused by death of brain neurons, which are the most important for information transmission in the brain nervous system, problems in the formation or function of synapses that transmit information between neurons, and abnormal symptoms or reduction in electrical activity of brain nerves. Examples of brain diseases include degenerative brain diseases. Degenerative brain diseases are aging-related diseases defined in association with progressive loss of specific neuronal populations and protein aggregates. Examples of degenerative brain diseases include stroke, apoplexy, dementia, Alzheimer's disease, Parkinson's disease, Huntington's disease, multiple sclerosis, and amyotrophic lateral sclerosis. A learning model may be constructed using the degree and progression of each of these diseases and the corresponding past detection signals as training data. Furthermore, by using the detection signal obtained this time and the situation related to brain activity, the accuracy of the learning model for the subject can be improved. For example, stages of progression of Alzheimer's disease include mild cognitive impairment (MCI), early stage, middle stage, and late stage.

The measurement information obtained by the signal analysis unit 7 is appropriately output. Examples of output include display on a monitor, printing on paper or the like, or output as electronic information to a terminal of a physician or the subject. In a preferred example of the brain activity measurement device 1, the device further comprises a wireless output unit 25 for wirelessly outputting the measurement information to outside of the subject. In particular, when the signal analysis unit 7 is expected to exist inside the brain of the subject or is physically connected to the subject, having such a wireless output unit 25 can facilitate the subject's daily life.

In a preferred example of the brain activity measurement device 1, at least one of the plurality of sheet-shaped probe units 9 has a second light source 21 having a wavelength different from the wavelength of the first light source 15. The second light source 21 is used for treating the brain of the subject. In this example, one of the plurality of sheet-shaped probe units 9 may have, for example, a second light control unit for controlling the output of the second light source 21. The second light control unit may be installed on the sheet-shaped probe unit 9. The second light control unit may have a computer or processor and control the output of the second light source 21. The second light control unit may also receive a control command from the control unit 20 and control the second light source 21 according to the control command. The control unit 20 may output information for controlling the second light source 21 to the second light control unit based on the measurement information obtained by the signal analysis unit 7. The expressions "second control unit" and "control unit 20" are functional, and they may be physically the same element. This brain activity measurement device 1 also functions as a treatment device for brain diseases by light. When the first light source 15 does not exist, the second light source 21 exists as a light source on the sheet-shaped probe 9.

In this example, the second light control unit or the control unit 20 stores the detection signal or measurement information related to brain activity and information related to the output of the second light source 21 (which may include information about which part of the second light source 21 to turn ON). The second light control unit or the control unit 20 may receive the detection signal or measurement information related to brain activity, read the information related to the output of the second light source 21 from the storage unit, and control the output of the second light source 21. The second light control unit or the control unit 20 may have constructed a learning model relating the detection signal or measurement information to the output of the second light source 21 and may use this learning model to obtain information for controlling the output of the second light source 21 from the detection signal or measurement information. By doing so, the brain can be appropriately treated based on the brain measurement information.

The brain activity measurement device 1 having the second light source 21 can be preferably used particularly when optogenes are introduced into the subject. Optogenetics refers to genes that can activate neurons based on light of a specific wavelength. Examples of photoactive proteins generated by introduction of these optogenes include channelrhodopsin-2, mutant ChR2, ChR2/H134R, ChR2/C128X (where X is T, A, or S) or ChR2/D156A, ChR2/E123T (ChETA), halorhodopsin, archaerhodopsin-3, archaerhodopsin T, OptoXRs, photoactivated adenylyl cyclase, and melanopsin. Methods for introducing optogenes that express these proteins are known.

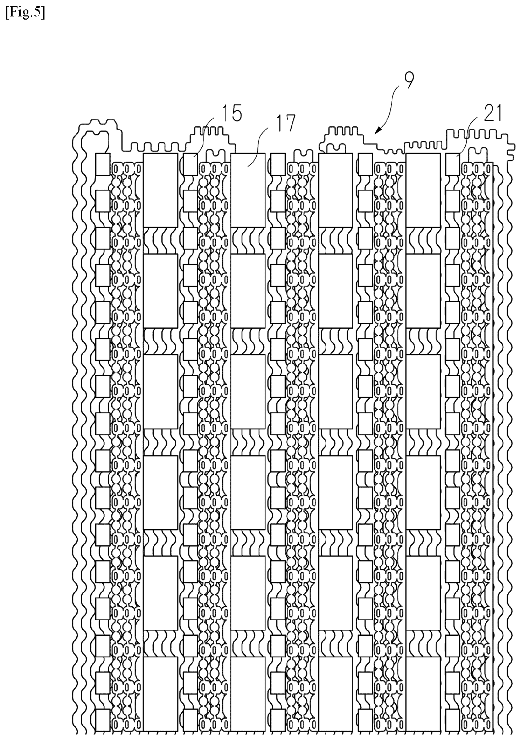

FIG. 5 is a conceptual diagram showing a design example of a circuit on a sheet-shaped probe unit having the first and second light sources. In the example shown in FIG. 5, a circuit board is formed on the sheet-shaped probe unit. The first light source 15, the first detector 17, and the second light source 21 are installed on the circuit board and connected by wiring.

In a preferred example of the brain activity measurement device 1, the signal analysis unit 7 analyzes brain activity of the subject using machine learning to obtain intention information of the subject. For example, when the subject is in a state where communication is difficult, such as being unable to speak, this example can be preferably used. In this example, a learning model is constructed using detection signals corresponding to each intention situation (grateful, happy, OK, NG, uncomfortable, want to stop). The signal analysis unit 7 may use such a learning model to obtain intention information of the subject based on the obtained detection signal. This brain activity measurement device 1 preferably further comprises an intention information output unit 23 for outputting the intention information. Examples of the intention information output unit 23 include a monitor attached to the subject and a monitor separate from the subject. When the intention information output unit 23 is a monitor attached to the subject, for example, icons corresponding to each intention situation (a smiling icon, an angry icon, etc.) may be displayed on the monitor. In this way, the intention of a subject who has difficulty communicating (for example, a person with advanced dementia or paralysis, or a mammal other than a human) can be displayed in an easily acceptable form.

In a preferred example of the brain activity measurement device 1, the signal analysis unit 7 analyzes activity of neurons of the subject using machine learning. For example, when the GECI described earlier is used, the activity of neurons of the subject can be analyzed.

In a preferred example of the brain activity measurement device 1, the device further comprises a deep brain stimulation electrode 31, a power supply unit 33 for supplying power to the deep brain stimulation electrode 31, and a supply power control unit 35 for controlling the power supplied by the power supply unit 33. The deep brain stimulation electrode is known, as described, for example, in Japanese Patent No. 6603656. The deep brain stimulation system using a deep brain stimulation electrode is also known, as described in Japanese Patent Publication No. 2008-513082. A preferred example of the brain activity measurement device 1 can be realized by appropriately applying these known technologies. For example, the supply power control unit 35 is configured to receive information from the control unit 20. The supply power control unit 35 may receive a control command from the control unit 20 and control the power supply unit 33 according to the control command. Since the control unit 20 receives measurement information, it may output a control command corresponding to the measurement information to the power supply unit 33. In this way, the deep brain stimulation electrode 31 can be driven in accordance with the brain activity state. The expressions "supply power control unit 35" and "control unit 20" are functional, and they may be physically the same element.

The brain activity measurement device 1 can be used by, for example, forming a hole 13 in the skull of the subject through surgery and laying a plurality of sheet-shaped probe units 9 on the surface of the brain parenchyma or the like via the hole 13.

### INDUSTRIAL APPLICABILITY

The present invention can be used in the field of medical devices related to the brain and in the field of communication tools for obtaining the intention of a subject.

### REFERENCE SIGNS LIST

1: Brain activity measurement device
3: Brain diagnostic probe
5: Detection signal receiving unit
7: Signal analysis unit
11: Skull of subject
13: Hole
15: First light source
17: First detector
19: First light control unit
20: Control unit
21: Second light source
23: Intention information output unit
25: Wireless output unit
31: Deep brain stimulation electrode
33: Power supply unit
35: Supply power control unit

## Claims

1. A brain activity measurement device comprising:
a brain diagnostic probe;
a detection signal receiving unit configured to receive a detection signal from the brain diagnostic probe; and
a signal analysis unit configured to analyze the detection signal received by the detection signal receiving unit and obtain measurement information for measuring brain activity,
wherein the brain diagnostic probe has a plurality of sheet-shaped probe units, each of the plurality of sheet-shaped probe units being configured to be placed inside a skull of a subject via a hole formed in the skull.

2. The brain activity measurement device according to claim 1,
wherein each of the plurality of sheet-shaped probe units has undulation corresponding to at least one of, or both, undulation of brain parenchyma and vascular structure at the site where each of the plurality of sheet-shaped probe units is laid.

3. The brain activity measurement device according to claim **1,**
wherein each of the plurality of sheet-shaped probe units has a shape configured to be laid on at least one of a subdural space, a subarachnoid space, a ventricular wall, or a brain sulcus of the subject.

4. The brain activity measurement device according to claim 1,
wherein the plurality of sheet-shaped probe units have a shape covering from 1% to 90%, inclusive, of the subject's brain region.

5. The brain activity measurement device according to claim 1,
wherein at least one of the plurality of sheet-shaped probe units comprises a first light source, a first detector, and a first light control unit configured to control light output from the first light source,
the first light source being used to excite a luminescent substance contained in the brain.

6. The brain activity measurement device according to claim 5,
wherein at least one of the plurality of sheet-shaped probe units further comprises a second light source having a wavelength different from a wavelength of the first light source,
the second light source being used for treating the brain of the subject.

7. The brain activity measurement device according to claim 1,
wherein the signal analysis unit analyzes brain activity of the subject using machine learning to obtain intention information of the subject,
and further comprises an intention information output unit configured to output the intention information.

8. The brain activity measurement device according to claim 1,
wherein the signal analysis unit analyzes activity of neurons of the subject by using machine learning.

9. The brain activity measurement device according to claim 8,
wherein the neurons of the subject emit light in response to a predetermined light stimulus by gene introduction or introduction of a luminescent substance.

10. The brain activity measurement device according to claim 1,
further comprising a wireless output unit configured to wirelessly output the detection signal or the measurement information to outside of the subject.

11. The brain activity measurement device according to claim 1,
further comprising a deep brain stimulation electrode, a power supply unit configured to supply power to the deep brain stimulation electrode, and a supply power control unit configured to control the power supplied by the power supply unit.
